# EUROPEAN PATENT APPLICATION

(11) **EP 4 202 028 A1**
(43) Date of publication of application: **28.06.2023**
(21) Application number: 21855946.6
(22) Date of filing: 06.08.2021
(51) Int. Cl.: C12M 1/00, C12M 1/34, C12N 15/09, C12Q 1/6844, G01J 1/02

(54) **NUCLEIC ACID AMPLIFICATION DEVICE, NUCLEIC ACID AMPLIFICATION METHOD, AND SAMPLE SOLUTION POSITION CONTROL METHOD**

(30) Priority: 11.08.2020 JP 2020135819
(71) Applicant: Kyorin Pharmaceutical Co., Ltd., Tokyo 101-8311 (JP)
(72) Inventor: GOHDA Keisuke, Uji-shi, Kyoto 611-0033 (JP)
(74) Representative: Handley, Matthew Edward
(86) International application number: PCT/JP2021/029406
(87) International publication number: WO 2022/034867

(57) **Abstract**

A nucleic acid amplification device, on which a nucleic acid amplification chip is placed, includes: temperature regulation units forming a denaturation temperature zone and an extension and annealing temperature zone; liquid delivery mechanisms that equalize air pressures of an air suction portion and an air discharge portion when liquid delivery is stopped; a detection unit that detects a sample solution in the nucleic acid amplification chip; and a control unit that controls the liquid delivery mechanisms based on a detection result of the detection unit. The detection unit includes an infrared light source that emits an infrared ray, and an infrared detection element that detects the infrared ray.

## Description

### Technical Field

The present disclosure relates to a nucleic acid amplification device, a nucleic acid amplification method, and a sample solution position control method.

### Background Art

Genetic testing is central to various fields such as clinical testing and the identification of types of crops or pathogenic microorganisms. The ability to detect various diseases including cancer, microbial infection, genetic markers based on molecular phylogenetic analysis, or the like has been a universal technique for disease and onset risk diagnosis, marker search, food or environment safety assessment, the proof of crime, and many other techniques.

One of the most powerful basic techniques of detecting a small amount of nucleic acid, which is a gene, with high sensitivity is a technique of analyzing a product obtained by exponentially replicating and amplifying a part or all of a nucleic acid sequence.

A PCR method is a powerful technique of selectively amplifying a specific region of DNA. In PCR, several millions of copies of DNA fragments can be generated from a single template DNA for a target DNA sequence in the template DNA. PCR repeats a three-phase or two-phase temperature condition called a thermal cycle. Accordingly, individual reactions such as denaturation of DNA into single strands, annealing of denatured DNA single strands and primers, and primer extension by a thermostable DNA polymerase enzyme are sequentially repeated. This cycle is repeated until a sufficient copy number is obtained for analysis. In principle, the copy number can be doubled in one cycle of PCR.

The PCR method is a powerful technique of exponentially amplifying a gene via a thermal cycle. However, in a general-purpose thermal cycler device used for PCR, temperature control is slow due to a very large heat capacity of an aluminum block portion that is a heater, and in the related art, 30 to 40 cycles of PCR operation requires one to two hours, or one to two hours or more according to circumstances. For this reason, even when a latest genetic testing device is used, a total of one hour or more is usually required for analysis. Therefore, speeding up the PCR operation has been a major issue since the advent of the technique.

A reciprocal flow type nucleic acid amplification device has been reported which uses a liquid delivery mechanism such as a microblower and which delivers a sample solution between two temperature zones in a reciprocating manner (Patent Literature 1).

### Citation List

### Patent Literature

Patent Literature 1: International Publication WO 2016/006612

### Summary of Invention

### Technical Problem

However, since the nucleic acid amplification device described in Patent Literature 1 confirms the passing of the sample solution using a fluorescence detector, and transmits an electric signal from the fluorescence detector to a liquid delivery control mechanism, the overall cost of the device is greatly affected. For this reason, the range of use of the nucleic acid amplification device may be limited. In addition, a nucleic acid amplification chip including a micro-flow channel has a limitation on the material to be used, such as selecting a material with low autofluorescence. As described above, expanding the range of use of the nucleic acid amplification device is required.

The present disclosure is conceived to solve such problems, and an object of the present disclosure is to provide a nucleic acid amplification device, a nucleic acid amplification method, and a sample solution position control method of which the range of use can be expanded.

### Solution to Problem

Here, as a result of intensive research, the inventors have found that a micro-flow channel can be irradiated with an infrared ray of a specific wavelength (for example, 1450 nm) and the passing of a sample solution can be confirmed by a change in the transmitted infrared ray. The inventors have completed the present disclosure based on the finding.

Namely, according to the present disclosure, there is provided a nucleic acid amplification device on which a nucleic acid amplification chip is placed, the device including: temperature regulation units forming a denaturation temperature zone and an extension and annealing temperature zone; liquid delivery mechanisms that equalize air pressures of an air suction portion and an air discharge portion when liquid delivery is stopped; a detection unit that detects a sample solution in the nucleic acid amplification chip; and a control unit that controls the liquid delivery mechanisms based on a detection result of the detection unit. The detection unit includes an infrared light source that emits an infrared ray, and an infrared detection element that detects the infrared ray.

In the nucleic acid amplification device according to the present disclosure, the denaturation temperature zone and the extension and annealing temperature zone can be formed in the nucleic acid amplification chip by placing the nucleic acid amplification chip, and by performing temperature regulation by means of the temperature regulation units. Therefore, the control unit can perform thermal cycling by controlling the liquid delivery mechanisms to reciprocate the sample solution between the temperature zones in the micro-flow channel of the nucleic acid amplification chip, based on a detection result of the detection unit. Here, the detection unit includes the infrared light source that emits an infrared ray, and the infrared detection element that detects the infrared ray. Therefore, the cost of the detection unit can be greatly reduced compared to the case of using a fluorescence detector, so that the overall cost of the device can also be greatly reduced. As a result, the nucleic acid amplification device can be widely used in each situation. Further, when the detection unit performs detection using an infrared ray, a fluorescent material is not required as with the fluorescence detector, so that limitation on the selection of a material for the nucleic acid amplification chip can be reduced. In addition, the fluorescence detector has sensitivity in the vicinity of a fluorescence wavelength of interest. Therefore, when a plurality of the fluorescence detectors are used, in order to avoid mutual influence of fluorescence intensities between different wavelengths, it is necessary to carefully consider the selection of fluorescence wavelengths and the disposition of the fluorescence detectors. On the other hand, the detectors using an infrared ray do not affect each other due to the mechanism in which the detectors detect light absorption at a specific position in the micro-flow channel. For this reason, a plurality of the detection units can be disposed without being restricted by a positional relationship therebetween. Since various restrictions can be reduced as described above, the range of use of the nucleic acid amplification device can be expanded.

According to the present disclosure, there is provided a nucleic acid amplification method using a thermal cycle in which a sample solution is reciprocated between a denaturation temperature zone and an extension and annealing temperature zone, the method including: a temperature regulation step of forming the denaturation temperature zone and the extension and annealing temperature zone; a liquid delivery step of moving the sample solution in a micro-flow channel so as to reciprocate between the denaturation temperature zone and the extension and annealing temperature zone by means of liquid delivery mechanisms that equalize air pressures of an air suction portion and an air discharge portion when liquid delivery is stopped; a detection step of detecting the sample solution in a nucleic acid amplification chip; and a control step of controlling the liquid delivery mechanisms based on a detection result of the detection step. In the detection step, an infrared light source irradiates the micro-flow channel with an infrared ray, and an infrared detection element detects the infrared ray that has transmitted through the micro-flow channel.

According to the nucleic acid amplification method, the same actions and effects as those of the nucleic acid amplification device described above can be obtained.

According to the present disclosure, there is provided a sample solution position control method including: a liquid delivery step of moving a sample solution in a micro-flow channel by means of liquid delivery mechanisms that equalize air pressures of an air suction portion and an air discharge portion when liquid delivery is stopped; an infrared detection step of irradiating the micro-flow channel with an infrared ray, and detecting the infrared ray that has transmitted through the micro-flow channel; and a position control step of controlling a position of the sample solution in the micro-flow channel by controlling a driving of the liquid delivery mechanisms based on a change in an amount of the infrared ray detected in the infrared detection step.

According to the sample solution position control method, when the position of the sample in the micro-flow channel is controlled, the driving of the liquid delivery mechanisms can be controlled based on a change in the amount of the infrared ray detected in the infrared detection step. In this case, since the detection step is executed using an infrared ray, the same effects as those of the nucleic acid amplification device described above can be obtained compared to the case of using a fluorescence detector. As described above, the range of use of the sample solution position control method can be expanded.

### Advantageous Effects of Invention

According to the present disclosure, it is possible to provide the nucleic acid amplification device, the nucleic acid amplification method, and the sample solution position control method of which the range of use can be expanded.

### Brief Description of Drawings

FIG. 1 is a schematic configuration diagram of a nucleic acid amplification device according to an embodiment of the present disclosure.
FIG. 2 is a plan view diagram of a nucleic acid amplification chip.
FIGS. 3(a) to 3(c) are schematic diagrams representing how a detection unit detects a sample solution moving in a micro-flow channel, and FIG. 3(d) is a graph (image) showing a change in the amount of an infrared ray detected by an infrared detection element in each situation of FIGS. 3(a) to 3(c).
FIG. 4 is a flowchart showing contents of a nucleic acid amplification method and a sample solution position control method according to the embodiment of the present disclosure.
FIG. 5 is a flowchart showing contents of the nucleic acid amplification method and the sample solution position control method according to the embodiment of the present disclosure.

### Description of Embodiments

Hereinafter, a nucleic acid amplification device according to one embodiment of the present disclosure will be described with reference to the accompanying drawings. Incidentally, in the description of the drawings, the same elements are denoted by the same reference signs, and duplicate descriptions will not be repeated.

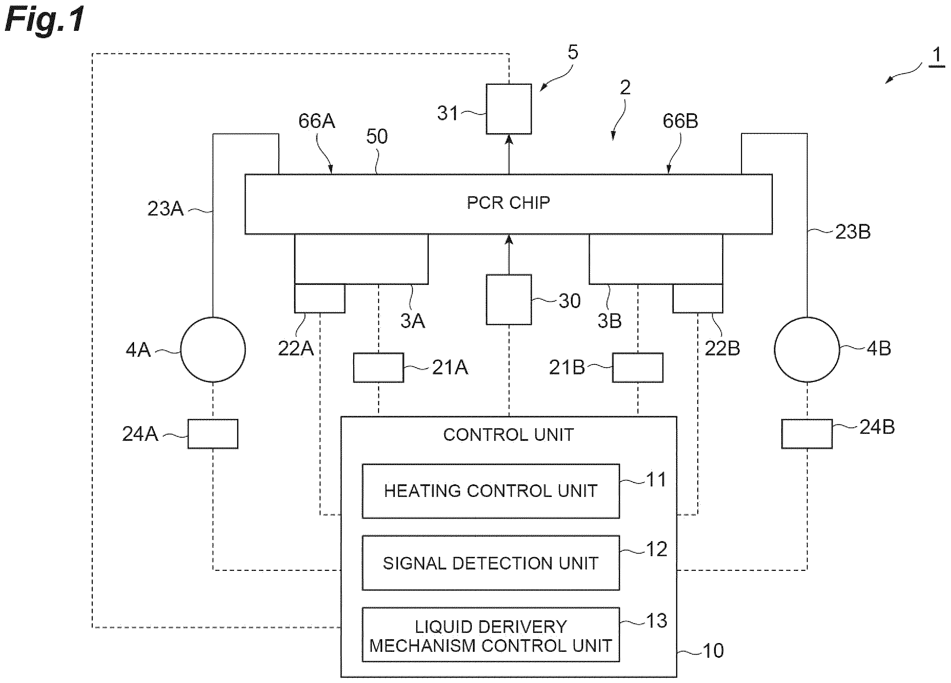

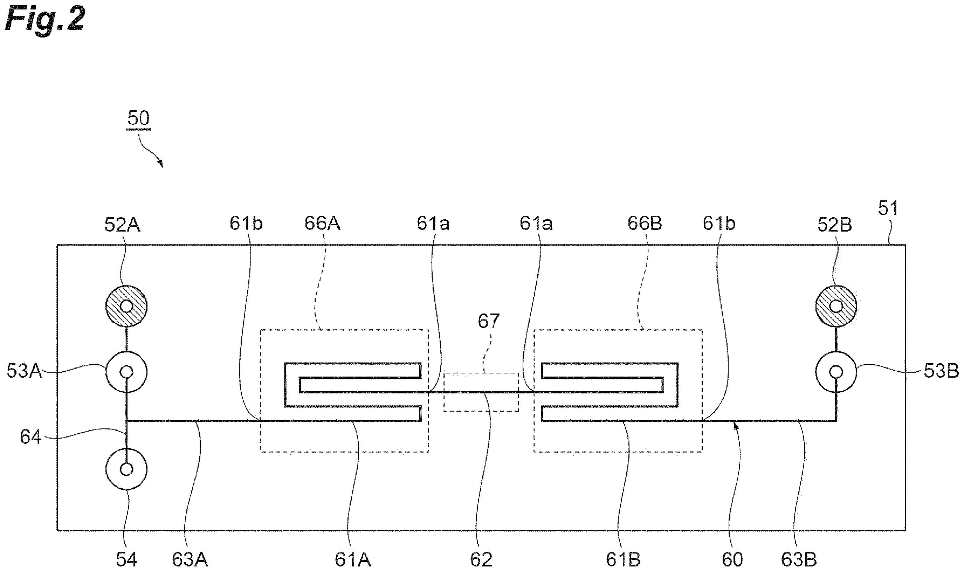

FIG. 1 is a schematic configuration diagram of a nucleic acid amplification device 1 according to an embodiment of the present disclosure. FIG. 2 is a plan view diagram of a nucleic acid amplification chip 50. The nucleic acid amplification device 1 according to the present embodiment is a device using a PCR method (polymerase chain reaction) in which thermal cycling is performed by placing the nucleic acid amplification chip 50 thereon and by reciprocating a sample solution between two temperature zones in a micro-flow channel of the nucleic acid amplification chip 50. The nucleic acid amplification device 1 performs nucleic acid amplification using a plurality of cycles of denaturation, annealing of primer pairs to opposite strands, and primer extension to exponential increase copy numbers of a target nucleic acid sequence. A nucleic acid amplification method using the nucleic acid amplification device 1 according to the present embodiment may use DNA as a template or may use RNA as a template. In the case of using RNA as a template, it is preferable that a reverse transcriptase is used together.

As shown in FIG. 1, the nucleic acid amplification device 1 includes a nucleic acid amplification chip placement portion 2, temperature regulation units 3A and 3B, liquid delivery mechanisms 4A and 4B, a detection unit 5, and a control unit 10.

The nucleic acid amplification chip placement portion 2 is a portion on which the nucleic acid amplification chip 50 is placed. The nucleic acid amplification chip placement portion 2 has a placement surface for placing the nucleic acid amplification chip 50. Incidentally, the placement surface of the nucleic acid amplification chip placement portion 2 may be formed of upper surfaces of the temperature regulation units 3A and 3B. Alternatively, a substrate for placing the nucleic acid amplification chip 50 on a heat source may be provided, and an upper surface of the substrate may be used as a placement surface.

Here, a configuration of the nucleic acid amplification chip 50 will be described with reference to FIG. 2. As shown in FIG. 2, the nucleic acid amplification chip 50 is configured by forming a micro-flow channel 60 in a chip member 51 having a plate shape.

It is preferable that the chip member 51 for forming the micro-flow channel 60 is made of a material satisfying all or some of requirements such as (i) having relatively high thermal conductivity, (ii) being stable in a temperature range required for PCR, (iii) being resistant to corrosion by electrolyte solutions or organic solvents, (iv) having low adsorption of nucleic acids or proteins. Specifically, examples of the material of the chip member 51 include glass, quartz, silicon, and various plastics such as cycloolefin polymer (COP), but are not limited thereto.

The micro-flow channel 60 is configured, for example, by forming a groove in a surface of the chip member 51 via a method such as machining such as cutting through NC processing, injection molding, nanoimprinting, or soft lithography, and by sealing the groove with a seal (preferably, for example, a transparent seal made of polyolefin or the like). Alternatively, the micro-flow channel 60 can be formed in the chip member 51 by 3D printing. The cross-sectional shape of the micro-flow channel 60 is not particularly limited and can be a semicircular shape, a circular shape, a rectangular shape, a trapezoidal shape, or the like. In addition, a cross section of the micro-flow channel 60 can have, for example, a width of approximately 10 to 1000 µm and a depth of approximately 10 to 1000 µm. In addition, each of the width and the depth of the micro-flow channel 60 can be constant, or the width or the depth can be partially changed.

In the example shown in FIG. 2, the micro-flow channel 60 includes heated portions 61A and 61B, an intermediate portion 62, connection portions 63A and 63B, and an introduction portion 64.

The heated portions 61A and 61B are portions that are heated by the temperature regulation units 3A and 3B (refer to FIG. 1) to regulate temperature of the passing sample solution. The heated portion 61A is formed in the chip member 51 at a position corresponding to a denaturation temperature zone 66A heated by the temperature regulation unit 3A. The heated portion 61B is formed in the chip member 51 at a position corresponding to an extension and annealing temperature zone 66B heated by the temperature regulation unit 3B. The shape of the heated portions 61A and 61B may be a shape that can ensure a flow channel length, namely, a region where the sample solution stays in each of the temperature zones 66A and 66B. For this reason, the shape of the heated portions 61A and 61B can be the shape of a serpentine flow channel having a loop shape or of a curved flow channel having a spiral shape or the like. Incidentally, the positions of the denaturation temperature zone 66A and the extension and annealing temperature zone 66B may be reversed.

The intermediate portion 62 is a portion that connects the heated portion 61A and the heated portion 61B. Namely, the heated portion 61A (denaturation temperature zone 66A) and the heated portion 61B (extension and annealing temperature zone 66B) are disposed apart from each other in a direction parallel to the surface of the chip member 51. Therefore, the intermediate portion 62 is disposed between the heated portion 61A and the heated portion 61B. In the example shown in FIG. 2, the intermediate portion 62 has a linearly extending shape, but may have a shape extending in a curved shape including a loop shape or the like. Incidentally, connection points between the heated portions 61A and 61B and the intermediate portion 62 are configured as entryways 61a and 61a of the temperature zones 66A and 66B.

The connection portions 63A and 63B are portions that connect communication ports 52A and 52B for the liquid delivery mechanisms 4A and 4B (refer to FIG. 1) and the heated portions 61A and 61B, respectively. Filters 53A and 53B are provided in the connection portions 63A and 63B, respectively. Incidentally, connection points between the heated portions 61A and 61B and the connection portions 63A and 63B are configured as entryways 61b and 61b of the temperature zones 66A and 66B, respectively. The introduction portion 64 is a portion that connects an introduction port 54 for the sample solution and the connection portion 63A. In addition, the introduction portion 64 may be provided to connect the introduction port 54 for the sample solution and the connection portion 63B. The introduction port 54 can be sealed by a seal, a valve, or the like, if necessary. Incidentally, the shapes of the connection portions 63A and 63B and the introduction portion 64 can be appropriately changed depending on a positional relationship and the like between the communication ports 52A and 52B and the introduction port 54, and are not limited to the shapes shown in FIG. 2.

Returning to FIG. 1, the temperature regulation units 3A and 3B form the denaturation temperature zone 66A and the extension and annealing temperature zone 66B, respectively. It is preferable that the temperature regulation units 3A and 3B maintain the denaturation temperature zone 66A and the extension and annealing temperature zone 66B at constant temperatures, respectively. In order to maintain such temperatures, it is preferable that the temperature regulation units 3A and 3B are formed of heat sources such as cartridge heaters. However, the temperature regulation units 3A and 3B are not particularly limited as long as the temperature regulation units 3A and 3B are temperatureregulatable devices.

Since the temperature regulation units 3A and 3B maintain temperatures of the denaturation temperature zone 66A and the extension and annealing temperature zone 66B at the constant temperatures, respectively, the heated portions 61A and 61B of the micro-flow channel 60 formed in the temperature zones 66A and 66B can also be maintained at corresponding temperatures. Therefore, the temperature regulation units 3A and 3B can change temperature of the sample solution that has moved to the temperature zones 66A and 66B, to desired temperatures in the temperature zones 66A and 66B, respectively.

For example, the temperature regulation unit 3Amay maintain the denaturation temperature zone 66A at a temperature required for the denaturation reaction of DNA in PCR. The temperature of the denaturation temperature zone 66A is preferably in a range of approximately 90 to 100°C, more preferably approximately 95°C. The temperature regulation unit 3B may maintain the extension and annealing temperature zone 66B at a temperature required for the annealing reaction and extension reaction of DNA in PCR. The temperature of the extension and annealing temperature zone 66B is preferably in a range of approximately 40 to 75°C, more preferably in a range of approximately 55 to 65°C.

Incidentally, the temperature regulation units 3A and 3B are connected to drivers 21A and 21B and to temperature monitoring units 22A and 22B, respectively. The drivers 21A and 21B are devices that control the temperature regulation units 3A and 3B to generate heat for maintaining the temperature zones 66A and 66B at the desired temperatures, based on control signals from the control unit 10. The temperature monitoring units 22A and 22B are devices that monitor temperatures of the temperature regulation units 3A and 3B to transmit monitoring results to the control unit 10.

The liquid delivery mechanisms 4A and 4B are mechanisms that move the sample solution in the micro-flow channel so as to reciprocate between the denaturation temperature zone 66A and the extension and annealing temperature zone 66B. As the liquid delivery mechanisms 4A and 4B, a mechanism is used which equalizes air pressures of an air suction portion and an air discharge portion when liquid delivery is stopped. Namely, as the liquid delivery mechanisms 4A and 4B, a mechanism configured to form an open system even during liquid delivery is used instead of using a mechanism requiring that the inside of the flow channel is a closed system. Since the liquid delivery mechanisms 4A and 4B are employed, when the liquid delivery mechanisms 4A and 4B stop blowing air, pressure inside the flow channel becomes equal to pressure outside the flow channel. Therefore, pressure acting on the sample solution having a plug shape is lost, so that liquid delivery is immediately stopped. For this reason, accurate position control can be performed even without a plurality of valves that release pressure to control the position of the sample solution. In such a manner, examples of the liquid delivery mechanisms 4A and 4B that equalize air pressures of the air suction portion and the air discharge portion when liquid delivery is stopped can include a microblower and a fan.

The micro-blower (also referred to as a piezoelectric microblower) provided as an example of the liquid delivery mechanisms 4A and 4B is a known device that suctions and discharges air. The microblower is characterized by not having a sealed structure, namely, not including a check valve. A typical microblower can realize the suction and discharge of air by bending and deforming a diaphragm via application of a voltage to a piezoelectric element. For example, a microblower manufactured by Murata Manufacturing Co., Ltd. (MZB1001T02 or MZB3004T04) can be used as the microblower. The fan provided as an example of the liquid delivery mechanisms 4A and 4B is a device that blows air via rotational motion of an impeller. A flow channel of the fan is not a closed system due to structural characteristics of the impeller.

The liquid delivery mechanisms 4A and 4B are connected to the communication ports 52A and 52B (refer to FIG. 2) of the nucleic acid amplification chip 50 via air supply flow channels 23A and 23B, respectively. The liquid delivery mechanisms 4A and 4B are connected to drivers 24A and 24B, respectively. The drivers 24A and 24B are devices that control the liquid delivery mechanisms 4A and 4B to supply air at a desired flow rate at a desired time, based on a control signal from the control unit 10. When the liquid delivery mechanism 4A supplies air, the sample solution in the micro-flow channel 60 (refer to FIG. 2) is delivered in a direction from a denaturation temperature zone 66A side toward an extension and annealing temperature zone 66B side. When the liquid delivery mechanism 4B supplies air, the sample solution in the micro-flow channel 60 (refer to FIG. 2) is delivered in a direction from the extension and annealing temperature zone 66B side toward the denaturation temperature zone 66A side.

Incidentally, in a case where the supply and suction of air are performed by one liquid delivery mechanism, the liquid delivery mechanism may perform liquid delivery while being connected to one communication port.

The detection unit 5 is a device that detects the sample solution in the nucleic acid amplification chip 50. The detection unit 5 detects the sample solution and transmits the detection result to the control unit 10. The detection unit 5 detects the sample solution between the denaturation temperature zone 66A and the extension and annealing temperature zone 66B in the nucleic acid amplification chip 50. Specifically, the detection unit 5 detects the sample solution in a detection region 67 in the vicinity of the center of the intermediate portion 62 of the micro-flow channel 60 (refer to FIG. 2).

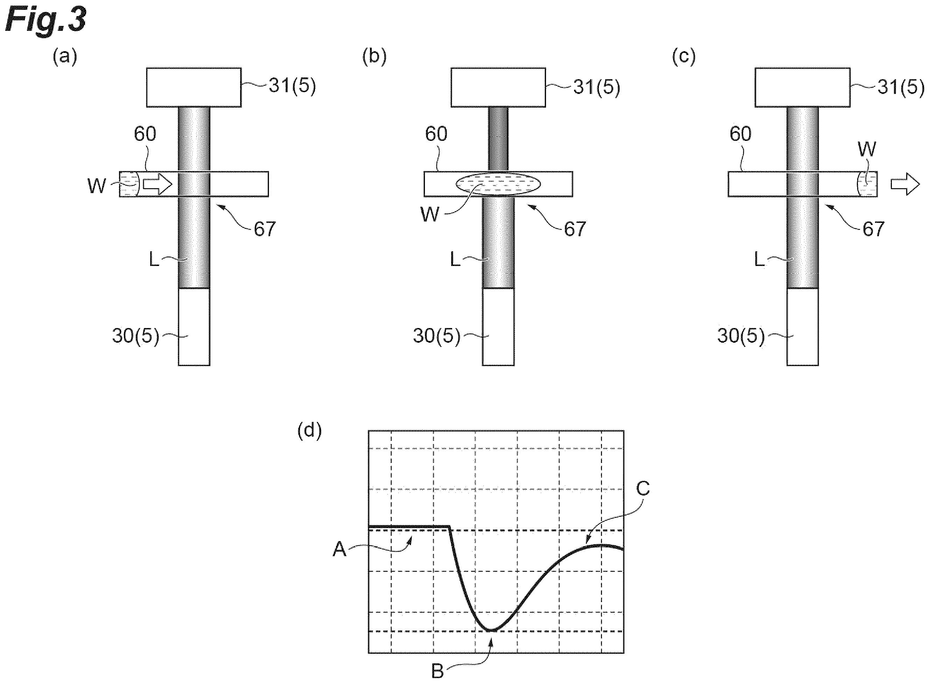

In the present embodiment, the detection unit 5 includes an infrared light source 30 that emits an infrared ray, and an infrared detection element 31 that detects the infrared ray. A sample solution detection method of the detection unit 5 will be described with reference to FIG. 3. FIGS. 3(a) to 3(c) are schematic diagrams showing a state where the detection unit 5 detects a sample solution W moving in the micro-flow channel 60. FIG. 3(d) is a graph (image) showing a change in the amount of an infrared ray detected by the infrared detection element 31 in each situation of FIGS. 3(a) to 3(c). As shown in FIGS. 3(a) to 3(c), the infrared light source 30 and the infrared detection element 31 are disposed to face each other with the micro-flow channel 60 interposed therebetween. The infrared light source 30 emits an infrared ray L to the infrared detection element 31 so as to pass through the detection region 67 of the micro-flow channel 60. The infrared detection element 31 detects the infrared ray (electromagnetic wave having a wavelength of 0.78 µm or more) that has transmitted through the micro-flow channel 60, converts the infrared ray into an electric signal, and transmits the electric signal to the control unit 10 (refer to FIG. 1).

Here, FIG. 3(a) shows the state of a stage before the sample solution W passes through the detection region 67 of the micro-flow channel 60. The amount of the infrared ray detected by the infrared detection element 31 at this time does not almost decrease as indicated by "A" in FIG. 3(d). FIG. 3(b) shows the state of a stage where the sample solution W passes through the detection region 67 of the micro-flow channel 60. In this state, the infrared ray L is absorbed by the sample solution W. The amount of the infrared ray detected by the infrared detection element 31 at this time decreases compared to in the region "A", as indicated by "B" in FIG. 3(d). FIG. 3(c) shows the state of a stage after the sample solution W has passed through the detection region 67 of the micro-flow channel 60. The amount of the infrared ray detected by the infrared detection element 31 at this time increases again as indicated by "C" in FIG. 3(d). From such a relationship, it is possible to detect that the sample solution W has passed through the detection region 67, based on the fact that the amount of the infrared ray detected by the infrared detection element 31 is a predetermined threshold value or less.

In the present embodiment, as the infrared light source 30, an infrared light emitting diode, an infrared laser light source, a xenon lamp, a tungsten lamp, a halogen lamp, or the like is employed. As the infrared light source 30, preferably, the infrared light emitting diode or the infrared laser light source is employed. The infrared light emitting diode is a diode that emits electromagnetic waves having a peak emission wavelength of 800 to 4400 nm. Examples of the infrared laser light source include a solid-state laser such as a fiber laser, a liquid laser, a gas laser, a semiconductor laser, and the like. A preferred peak emission wavelength of the infrared light emitting diode or the infrared laser light source is 920 to 970 nm, 1300 to 1550 nm, or 1900 to 2000 nm, and a more preferred peak emission wavelength is 1400 to 1500 nm.

Examples of the infrared light source 30 of a preferred peak emission wavelength can include an infrared light emitting diode or a laser light source having a peak emission wavelength of 950 nm, 965 nm, 970 nm, 1450 nm, 1950 nm, 2000 nm, or 3300 nm. Specific examples of the infrared light source 30 can include EOLS-950-843 (KLV Co., Ltd.), EOLS-965-525 (KLV Co., Ltd.), EOLS-970-195 (KLV Co., Ltd.), EOLD-1450-015 (KLV Co., Ltd.), EOLS-1450-995 (KLV Co., Ltd.), EOLS-1450-199 (KLV Co., Ltd.), EOLS-1450-843 (KLV Co., Ltd.), EOLC-1450-17-1 (KLV Co., Ltd.), L13895-0145P (Hamamatsu Photonics K.K.), L13895-0145G (Hamamatsu Photonics K.K.), L10660 (Hamamatsu Photonics K.K.), L 10660-01 (Hamamatsu Photonics K.K.), OIS-150-1450p-X-T (OSA Opto Light GmbH), KEDE1452H (KYOTO SEMICONDUCTOR Co., Ltd.), KEDE1454H (KYOTO SEMICONDUCTOR Co., Ltd.), KEDE1458K (KYOTO SEMICONDUCTOR Co., Ltd.), FPL1940S (Thorlabs Japan Inc.), FPL2000S (Thorlabs Japan Inc.), FPL2000C (Thorlabs Japan Inc.), L13771-0330C (Hamamatsu Photonics K.K.), and L13771-0330M (Hamamatsu Photonics K.K.).

The infrared detection element 31 is roughly classified into a thermal detection element having no wavelength dependence and a quantum detection element having wavelength dependence, depending on the principle of operation.

The thermal detection element is an element that converts a change in the temperature of a light receiving surface caused by incidence of radiant energy, into an electric signal. Examples of the thermal detection element include a thermopile that is a thermoelectromotive force detection element in which thermocouples are connected in series to increase a thermoelectromotive force, a thermistor bolometer that is a thermal conduction detection element using a change in resistance caused by heat, and a pyroelectric element that is made of a ferroelectric material and that uses the pyroelectric effect in which an electric charge is generated by spontaneous polarization occurring when the temperature of the ferroelectric material rises.

The quantum detection element is an element that obtains an electric signal proportional to the number of protons when the incident photons directly interact with electrons. As the quantum detection element, photoelectron emission (photomultiplier tube or image converter), a photovoltaic element (photodiode), a photoconductive element (photoconductive detector, photoconductive detector), photoelectromagnetic effect (photoelectromagnetic detector: PEM detector), or the like is used. Incidentally, one example of the quantum detection element is KPDE086S-H8 manufactured by KYOTO SEMICONDUCTOR Co., Ltd..

Here, the quantum detection element may be preferable to the thermal detection element when used as the infrared detection element 31 of the detection unit 5 of the nucleic acid amplification device 1. Specifically, the reason is that the quantum detection element has the advantage of a high response speed.

The control unit 10 may be configured as, for example, a computer including a processor, a memory, a storage, and a communication interface. The processor is a computational unit such as a central processing unit (CPU). The memory is a storage unit such as a read only memory (ROM) or a random access memory (RAM). The storage is a storage unit (storage medium) such as a hard disk drive (HDD). The communication interface is a communication device that realizes data communication. The processor controls the memory, the storage, and the communication interface to realize the function of the control unit 10 to be described later. In the control unit 10, for example, various functions are realized by loading a program stored in the ROM into the RAM, and by causing the CPU to execute the program loaded in the RAM. The number of the computers forming the control unit 10 may be one or two or more.

The control unit 10 includes a heating control unit 11, a signal detection unit 12, and a liquid delivery mechanism control unit 13. The heating control unit 11 controls the temperature regulation units 3A and 3B such that the temperatures of the denaturation temperature zone 66A and the extension and annealing temperature zone 66B are kept constant at the desired temperatures. The signal detection unit 12 receives a detection result from the detection unit 5, namely, an electric signal corresponding to the amount of the infrared ray detected by the infrared detection element 31. In addition, the signal detection unit 12 identifies the amount of the infrared ray from the received electric signal, and detects the presence of the sample solution in the detection region 67 (refer to FIGS. 2 and 3) based on the degree of decrease in the amount of the infrared ray.

The liquid delivery mechanism control unit 13 controls the liquid delivery mechanisms 4A and 4B to move the sample solution in the micro-flow channel 60 as desired. The liquid delivery mechanism control unit 13 controls the position of the sample solution in the micro-flow channel 60 by controlling the driving of the liquid delivery mechanisms 4A and 4B based on a change in the amount of the infrared ray detected by the signal detection unit 12. For example, the liquid delivery mechanism control unit 13 may perform position control to stop the supply of air by the liquid delivery mechanism 4A or the liquid delivery mechanism 4B, and to stop the sample solution in that place at the time a certain standby time has elapsed after the detection of the sample solution by the detection unit 5.

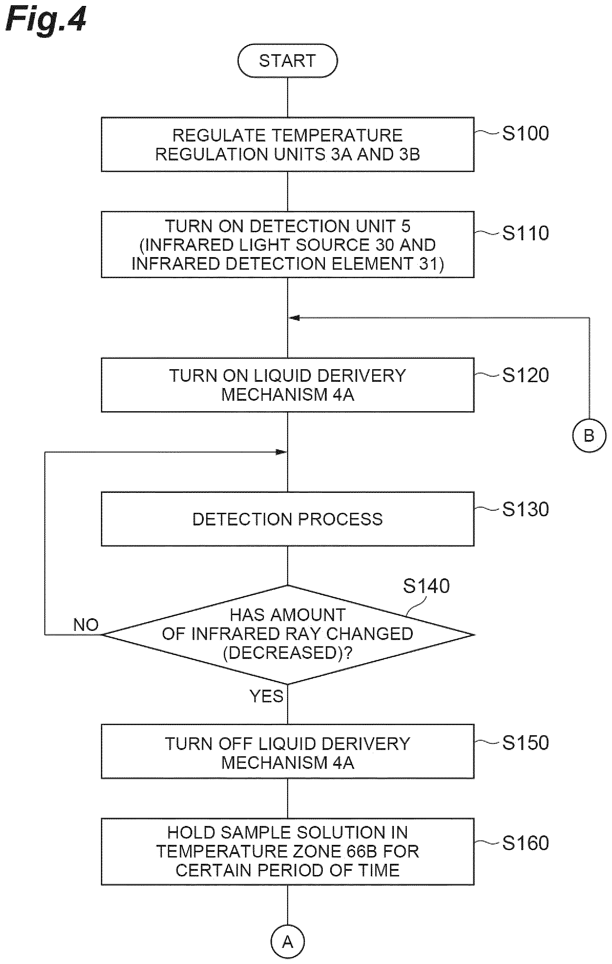

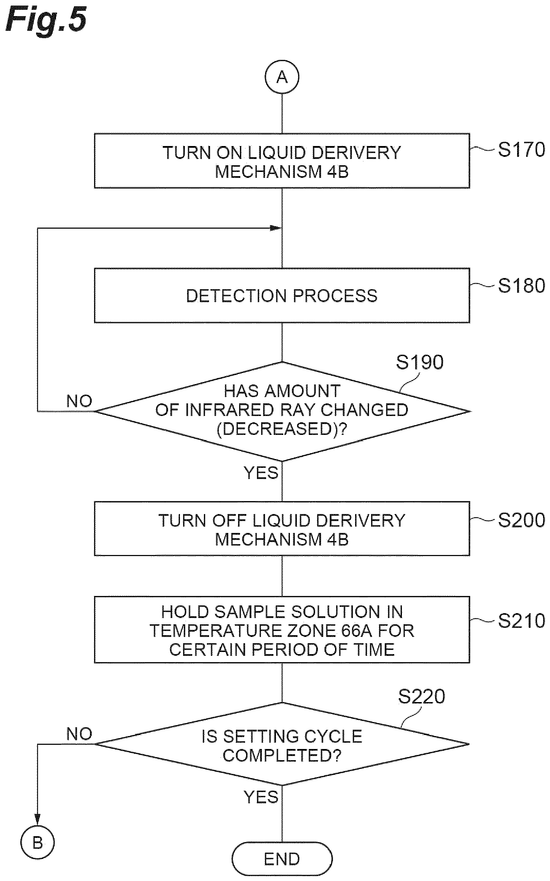

Next, contents of a nucleic acid amplification method and a sample solution position control method according to the embodiment of the present disclosure will be described with reference to FIGS. 4 and 5. FIGS. 4 and 5 are flowcharts showing the contents of the nucleic acid amplification method and the sample solution position control method according to the embodiment of the present disclosure. The processes shown in FIGS. 4 and 5 are repeatedly executed in the control unit 10 at predetermined times.

As shown in FIG. 4, the heating control unit 11 performs a temperature regulation process by controlling the temperature regulation units 3A and 3B to form the denaturation temperature zone 66A and the extension and annealing temperature zone 66B (step S100: temperature regulation step). Next, the infrared light source 30 is turned on and the infrared detection element 31 is allowed to measure the amount of an infrared ray by turning on the detection unit 5 (step S110: detection unit adjustment step). Next, the liquid delivery mechanism control unit 13 performs a liquid delivery process for moving the sample solution in the micro-flow channel 60 from the denaturation temperature zone 66A to the extension and annealing temperature zone 66B by turning on the liquid delivery mechanism 4A (step S120: liquid delivery step 1). Next, the signal detection unit 12 performs a detection process for detecting the sample solution in the nucleic acid amplification chip by causing the infrared light source 30 to irradiate the micro-flow channel 60 with an infrared ray, and by causing the infrared detection element 31 to detect the infrared ray that has transmitted through the micro-flow channel 60 (step S130: infrared detection step 1).

Next, the control unit 10 determines whether or not there is a change (decrease) in the amount of the infrared ray (step S140). In step S140, when it is determined that the sample solution is detected in step S130 and it is time to stop the liquid delivery mechanism A (YES in step S140), the liquid delivery mechanism control unit 13 turns off and stops the liquid delivery mechanism 4A based on a detection result (change in the amount of the detected infrared ray) in step S130 (step S150: position control step 1). Next, the liquid delivery mechanism control unit 13 causes the sample solution to be held in the extension and annealing temperature zone 66B for a certain period of time (step S160). Incidentally, when the determination is NO in step S140, the processes are repeated from step S130 again.

Next, as shown in FIG. 5, the liquid delivery mechanism control unit 13 performs a liquid delivery process for moving the sample solution in the micro-flow channel 60 from the extension and annealing temperature zone 66B to the denaturation temperature zone 66A by turning on the liquid delivery mechanism 4B (step S170: liquid delivery step 2). Next, the signal detection unit 12 performs a detection process for detecting the sample solution in the nucleic acid amplification chip by causing the infrared light source 30 to irradiate the micro-flow channel 60 with an infrared ray, and by causing the infrared detection element 31 to detect the infrared ray that has transmitted through the micro-flow channel 60 (step S180: infrared detection step 2). Next, the control unit 10 determines whether or not there is a change (decrease) in the amount of the infrared ray (step S190).

In step S190, when it is determined that the sample solution is detected in step S 180 and it is time to stop the liquid delivery mechanism B (YES in step S 190), the liquid delivery mechanism control unit 13 turns off and stops the liquid delivery mechanism 4B based on a detection result (change in the amount of the detected infrared ray) in step S180 (step S200: position control step 2). Next, the liquid delivery mechanism control unit 13 causes the sample solution to be held in the denaturation temperature zone 66A for a certain period of time (step S210). Incidentally, when the determination is NO in step S190, the processes are repeated from step S 180 again.

Next, the control unit 10 determines whether or not a setting cycle is completed (step S220). When the determination is NO in step S220, the processes are repeated from step S 120 of FIG. 4 again. When the determination is YES in step S220, the processes shown in FIGS. 4 and 5 are ended. After the processes shown in FIGS. 4 and 5 have ended, the processes are restarted from step S100, if necessary.

One example of operation when PCR testing is performed using the nucleic acid amplification device 1 according to the present embodiment will be described. In a case where PCR testing is performed by the nucleic acid amplification device 1 using an infrared ray, the following is an example of operation that cannot be performed by a nucleic acid amplification device using a fluorescence detector. First, a length of the sample solution in the micro-flow channel can be accurately measured by disposing a plurality of detectors side by side in close proximity. In addition, a flow velocity in a short section can be measured using two detectors disposed in close proximity, and a flow velocity change in the micro-flow channel can be measured by preparing a plurality of the detectors. A control system that accurately stops the sample solution at any position in the micro-flow channel can be realized using the result.

Next, actions and effects of the nucleic acid amplification device 1, the nucleic acid amplification method, and the sample solution position control method according to the present embodiment will be described.

In the nucleic acid amplification device 1 according to the present embodiment, the denaturation temperature zone 66A and the extension and annealing temperature zone 66B can be formed in the nucleic acid amplification chip 50 by placing the nucleic acid amplification chip 50, and by performing temperature regulation by means of the temperature regulation units 3A and 3B. Therefore, the control unit 10 can perform thermal cycling by controlling the liquid delivery mechanisms 4A and 4B to reciprocate the sample solution between the temperature zones 66A and 66B in the micro-flow channel 60 of the nucleic acid amplification chip 50, based on a detection result of the detection unit 5. Here, the detection unit 5 includes the infrared light source 30 that emits an infrared ray, and the infrared detection element 31 that detects the infrared ray. Therefore, the cost of the detection unit 5 can be greatly reduced compared to the case of using a fluorescence detector, so that the overall cost of the device can also be greatly reduced. Therefore, the nucleic acid amplification device 1 can be widely used in each situation.

Further, when the detection unit 5 performs detection using an infrared ray, there is no need to select a material with low autofluorescence as when the fluorescence detector is used, so that limitation on the selection of a material for the nucleic acid amplification chip 50 can be reduced. For example, PMMA, PP, and other opaque (since the wavelength of an infrared ray transmits through the most of resins) materials can also be used by using an infrared ray although they cannot be employed as the material of the chip member 51 in the case of using the fluorescence detector. This means that using an infrared ray is useful to the sample solution that is degraded by visible light.

In addition, since the detection unit 5 using an infrared ray does not use a detection method involving light emission as with the fluorescence detector, a plurality of the detection units 5 can be disposed without being restricted by a positional relationship therebetween. For example, in the micro-flow channel 60 shown in FIG. 2, instead of disposing the detection unit 5 in the detection region 67, the additional detection unit 5 may be disposed in the vicinity of each of the entryways 61a and 61a or the entryways 61b and 61b of the heated portions 61A and 61B. Alternatively, the plurality of detection units 5 may be disposed in the intermediate portion 62. In such a manner, even when the detection units 5 are disposed close to each other, unlike fluorescence of the fluorescence detector, an infrared ray from one detection unit 5 do not adversely affect detection information of the other detection unit 5. In addition, the detection units 5 may be disposed in the vicinities of the entryways 61a and 61a or the entryways 61b and 61b of the heated portions 61A and 61B in addition to in the detection region 67. In addition, for example, detection by the detection unit 5 may be performed above heaters (heated portions 61A and 61B) by forming microholes in the heaters of the temperature regulation units 3A and 3B.

Since various restrictions can be reduced as described above, the range of use of the nucleic acid amplification device can be expanded.

According to the present embodiment, there is provided a nucleic acid amplification method using a thermal cycle in which a sample solution is reciprocated between a denaturation temperature zone 66A and an extension and annealing temperature zone 66B, the method including: a temperature regulation step of forming the denaturation temperature zone 66A and the extension and annealing temperature zone 66B; a liquid delivery step of moving the sample solution in a micro-flow channel 60 so as to reciprocate between the denaturation temperature zone 66A and the extension and annealing temperature zone 66B by means of liquid delivery mechanisms 4A and 4B that equalize air pressures of an air suction portion and an air discharge portion when liquid delivery is stopped; a detection step of detecting the sample solution in a nucleic acid amplification chip 50; and a control step of controlling the liquid delivery mechanisms based on a detection result of the detection step. In the detection step, an infrared light source irradiates the micro-flow channel 60 with an infrared ray, and an infrared detection element detects the infrared ray that has transmitted through the micro-flow channel 60.

According to the nucleic acid amplification method, the same actions and effects as those of the nucleic acid amplification device 1 described above can be obtained.

According to the present embodiment, there is provided a sample solution position control method including: a liquid delivery step of moving a sample solution in a micro-flow channel 60 by means of liquid delivery mechanisms 4A and 4B that equalize air pressures of an air suction portion and an air discharge portion when liquid delivery is stopped; an infrared detection step of irradiating the micro-flow channel 60 with an infrared ray, and detecting the infrared ray that has transmitted through the micro-flow channel 60; and a position control step of stopping the sample solution in the micro-flow channel 60 at a predetermined position by controlling a driving of the liquid delivery mechanisms 4A and 4B based on a change in an amount of the infrared ray detected in the infrared detection step.

According to the sample solution position control method, when the position of the sample in the micro-flow channel 60 is controlled, the driving of the liquid delivery mechanisms 4A and 4B can be controlled based on a change in the amount of the infrared ray detected in the infrared detection step. In this case, since the detection step is executed using an infrared ray, the same effects as those of the nucleic acid amplification device 1 described above can be obtained compared to the case of using a fluorescence detector. As described above, the range of use of the sample solution position control method can be expanded.

The present disclosure is not limited to the above-described embodiment.

For example, the shape of the micro-flow channel shown in FIG. 2, the positional relationship, and the like between the components of the nucleic acid amplification chip, or the like is merely one example, and may be changed as appropriate.

In addition, the configuration of the nucleic acid amplification device shown in FIG. 1 is merely one example, and the positional relationship between the components may be changed as appropriate, some of the components may be omitted, or other components may be added.

### Reference Signs List

1: nucleic acid amplification device, 3A, 3B: temperature regulation unit, 4A, 4B: liquid delivery mechanism, 5: detection unit, 10: control unit, 30: infrared light source, 31: infrared detection element, 50: nucleic acid amplification chip, 66A: denaturation temperature zone, 66B: extension and annealing temperature zone.

## Claims

1. A nucleic acid amplification device on which a nucleic acid amplification chip is placed, the device comprising:
temperature regulation units forming a denaturation temperature zone and an extension and annealing temperature zone;
liquid delivery mechanisms that equalize air pressures of an air suction portion and an air discharge portion when liquid delivery is stopped;
a detection unit that detects a sample solution in the nucleic acid amplification chip; and
a control unit that controls the liquid delivery mechanisms based on a detection result of the detection unit,
wherein the detection unit includes an infrared light source that emits an infrared ray, and an infrared detection element that detects the infrared ray.

2. A nucleic acid amplification method using a thermal cycle in which a sample solution is reciprocated between a denaturation temperature zone and an extension and annealing temperature zone, the method comprising:
a temperature regulation step of forming the denaturation temperature zone and the extension and annealing temperature zone;
a liquid delivery step of moving the sample solution in a micro-flow channel so as to reciprocate between the denaturation temperature zone and the extension and annealing temperature zone by means of liquid delivery mechanisms that equalize air pressures of an air suction portion and an air discharge portion when liquid delivery is stopped;
a detection step of detecting the sample solution in a nucleic acid amplification chip; and
a control step of controlling the liquid delivery mechanisms based on a detection result of the detection step,
wherein in the detection step, an infrared light source irradiates the micro-flow channel with an infrared ray, and an infrared detection element detects the infrared ray that has transmitted through the micro-flow channel.

3. A sample solution position control method comprising:
a liquid delivery step of moving a sample solution in a micro-flow channel by means of liquid delivery mechanisms that equalize air pressures of an air suction portion and an air discharge portion when liquid delivery is stopped;
an infrared detection step of irradiating the micro-flow channel with an infrared ray, and detecting the infrared ray that has transmitted through the micro-flow channel; and
a position control step of controlling a position of the sample solution in the micro-flow channel by controlling a driving of the liquid delivery mechanisms based on a change in an amount of the infrared ray detected in the infrared detection step.
